# EUROPEAN PATENT APPLICATION

(11) **EP 1 616 958 A2**
(43) Date of publication of application: **18.01.2006**
(21) Application number: 05076507.2
(22) Date of filing: 06.11.1997
(51) Int. Cl.: C12N 15/63, C12N 15/85, C12N 5/18, C07K 14/705, C07K 16/28, C07K 19/00, G01N 33/50, G01N 33/53, A61K 38/17

(54) **Notch-ligands for use in immunotherapy**

(30) Priority: 07.11.1996 GB 9623236; 24.07.1997 GB 9715674; 11.09.1997 GB 9719350
(62) Divisional of application: 97911353.7
(71) Applicant: Lorantis Limited, Cambridge CB4 0PE (GB)
(72) Inventor: Lamb, Jonathan Robert The University of Edinburgh, Edinburgh EH8 9AG (GB); Dallman, Margaret Jane Imperial College of Science, London SW7 2AZ (GB); Hoyne, Gerard Francis Medical Genome Centre, PO Box 334, Canberra, ACT (AU)
(74) Representative: Mallalieu, Catherine Louise

(57) **Abstract**

The present invention relates to the use of therapeutic compounds in the modification of T-cell, T-cell-antigen presenting cell (APC) interactions and the interactions between pathogenic organisms and immunocompetent cells of a host. In particular it relates to the use of these compounds in the modulation of the interaction between *Notch* proteins and their ligands and to the use of such compounds in the therapy of conditions such as graft rejection, autoimmunity, allergy, and asthma and infectious diseases.

## Description

The present invention relates to use of therapeutic compounds in the modification of T-cell activation. In particular it relates to their use in modulating the interaction between *Notch* protein family members and their ligands and to the use of such compounds in the therapy of conditions such as graft rejection, autoimmunity, allergy, asthma, infectious diseases and tumours.

The controlled interaction between T cells and between antigen presenting cells (APC) and T cells is vital to the function of the human immune system. However in certain pathological states it may be therapeutically beneficial to modify, positively or negatively, such interactions. For example, in conditions such as autoimmunity, allergy and graft rejection it is desirable to induce the downregulation of an immune response by stimulation of negative T cell or T cell-APC interaction. Models of "infectious tolerance" and "linked suppression" suggest that tolerance may be induced in a small number of T cells and that these T cells then transmit this tolerance to other T cells thus preventing an effective immunological attack. In other pathological conditions such as tumour induced immunosuppression, parasitic viral or bacterial infections, immunosuppression is a common feature. In such circumstances it would therefore be desirable to inhibit the T cell interactions passing on the infectious tolerance.

However until now the mechanisms underlying such T cell and T cell-APC interactions have not been understood.

WO 92/19734 purports to disclose the nucleotide sequences of the human *Notch* and *Delta* genes and amino acid sequences of their encoded proteins. The disclosure shows that the *Notch* gene family has been well characterised as essential to the correct embryological cell lineage development of insects such as Drosophila.

Proteins belonging to the *Notch* family are transmembrane receptors that contain several conserved peptide motifs. Each protein within the family displays characteristic extracellular EGF (epidermal growth factor)-like repeats and a juxtamembrane Lin-12/*Notch* motif. In addition each protein has 6-8 ankyrin repeat motifs on the cytoplasmic tail together with a PEST sequence. The *Notch* ligands have a diagnostic DSL domain (D.*Delta*, S, *Serrate*, L,Lag2) comprising 20-22 amino acids at the amino terminus of the protein and between 3-8 EGF-like repeats on the extracellular surface. The proteins have a short cytoplasmic tail with no conserved functional domains.

Recent evidence suggests that *Notch* signalling contributes to lineage commitment of immature T cells in the thymus, biasing thymocyte development towards the CD8+ lineage which is independent of MHC recognition (Robey E, et al. Cell 1996, 87:483-492). During maturation in the thymus, T cells acquire the ability to distinguish self antigens from those that are non-self, a process termed self tolerance (von Boehmer H, et al. Ann Rev Immunol. 1990;8:531). Mechanisms also exist in the periphery for the induction and maintenance of tolerance and in many respects their importance is under estimated. There are many experimental models of graft rejection, autoimmune disease and specific responses to allergens that clearly illustrate the ability to induce a state of specific unresponsiveness (tolerance or anergy) in the recipient by immunisation with an antigen. From these systems two important findings arise. Firstly, immunisation with a peptide fragment of antigen under selected conditions may inhibit specific responses not only to itself but to other regions in the same molecule provided the intact protein is used for the challenge immunisation (linked suppression; Hoyne GF, et al. J. Exp Med. 1993; 178:183. and Metzler B, Wraith DC. Int. Immunol. 1993;5:1159). Secondly, as best described in experimental models of transplantation is the phenomenon of "infectious tolerance" where it is postulated that immunocompetent cells made tolerant to a specific antigen are able to inhibit other cells from responding and further, that this second population of cells becomes regulatory and tolerant (Qin SX, et al. Science 1993;258:974). The immunological mechanisms underlying these phenomena have not so far been characterised.

The present invention arises from the discovery that the *Notch* receptor family and its ligands, *Delta* and *Serrate,* are expressed on the cell surface of normal adult cells of the peripheral immune system.

Hence there is provided according to the present invention, the use of a *Notch-*ligand in the manufacture of a medicament for use in immunotherapy.

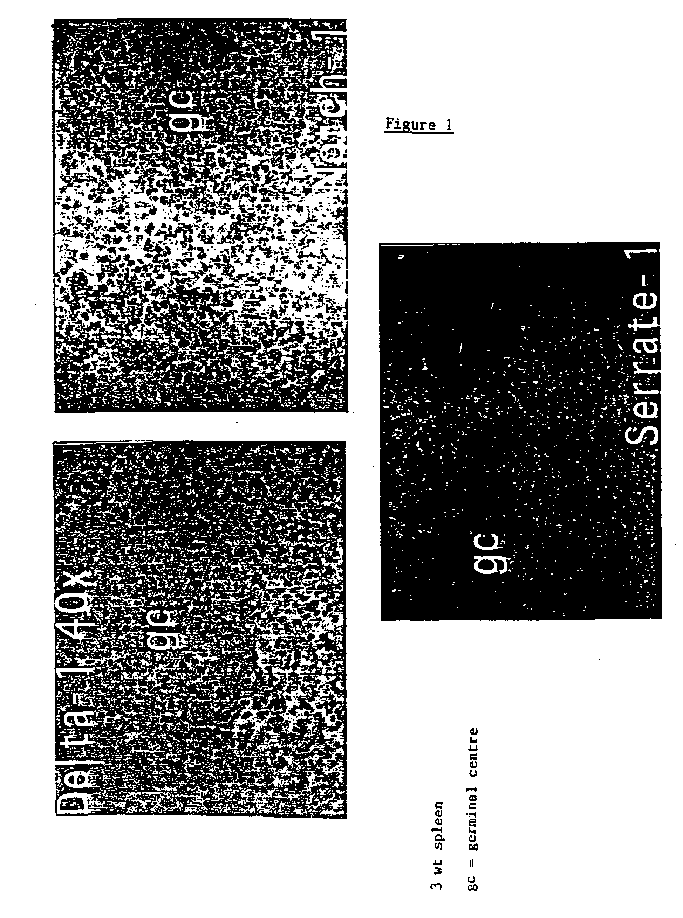

The expression pattern of the *Notch* family of receptors and their ligands in the normal peripheral adult immune system has not previously been described but the present inventors have shown that T cells express *Notch* 1 mRNA constitutively, while *Delta* expression is limited to only a subset of T cells in the peripheral lymphoid tissues. *Serrate* expression appears restricted to a subset of antigen presenting cells (APCs) in the periphery (Figure 1). Hence this receptor ligand family may continue to regulate cell fate decisions in the immune system as has been shown in other tissues, beyond embryonic development (Ellisen LW, et al. Cell 1991;66:649). *Notch* signalling may play a central role in the induction of peripheral unresponsiveness (tolerance or anergy) and may provide a physical explanation for linked suppression and infectious tolerance.

The present invention further relates to the use of a *Notch*-ligand in the treatment of a T cell mediated reaction. Thus, it has been observed that by exposing a population of naive T cells to a *Notch*-ligand expressed by an APC in the presence of an allergen or antigen, the Notch-ligand is capable of making the T cell population tolerant to said allergen or antigen. Furthermore, this T cell population, when subsequently exposed to a second population of naive T cells is capable of rendering the second population tolerant to said allergen or antigen.

Thus, the invention also relates to the use of a *Notch*-ligand in affecting linked tolerance and/or bystander tolerance (also known in the art as infectious tolerance).

A further embodiment of the invention relates to the use of a *Notch*-ligand in the modulation of the expression of a functional *Notch*-protein or *Notch* signalling pathways.

In the above described embodiments of the present invention, the *Notch*-ligand may be exposed to the T cells by incubating/mixing the T cells with antigen presenting cells (APCs) or the like, that express or overexpress a *Notch*-ligand in the presence of an allergen or antigen. The (over)expression of the *Notch*-ligand gene may be brought about by the APCs being transfected with a gene capable of expressing a *Notch*-ligand or by the APCs being exposed to an agent capable of up-regulating expression of endogenous *Notch-ligand* gene(s).
Suitable agents that influence expression of *Notch*-ligands include agents that affect the expression of Delta and/or Serrate genes. For instance, for Delta expression, binding of extracellular BMPs (bone morphogenetic proteins, Wilson, P.A. and Hemmati-Brivanlou A.1997 Neuron 18: 699-710, Hemmati-Brivanlou, A and Melton, D. 1997 Cell 88:13-17) to their receptors leads to down-regulated Delta transcription due to the inhibition of the expression of the Achaete/Scute Complex transcription factor. This complex is believed to be directly involved in the regulation of Delta expression. Thus, any agent that inhibits the binding of BMPs to their receptors is capable of producing an increase in the expression of Delta and/or Serrate. Such agents include Noggin, Chordin, Follistatin, Xnr3, FGFs, Fringe and derivatives and variants thereof (see references for noggin-Smith W. C. and Harland, R.M Cell 70:829-840, chordin-Sasai, Yet al., 1994 Cell 79: 779-790). Noggin and Chordin bind to BMPs thereby preventing activation of their signalling cascade which leads to decreased Delta transcription.

In some disease states, the body may be immuno-compromised and it may be desirable to downregulate the expression of Delta and/or Serrate in order to overcome the imposed immunosuppression. Agents that inhibit the expression of Delta and/or Serrate may be used in such circumstances. An example of agents that inhibit the expression of Delta and/or Serrate include the Toll protein (Medzhitov, R.et al., 1997 Nature 388: 394-397) BMPs and other agents that decrease or interfere with the production of Noggin, Chordin, Follistatin, Xnr3, FGFs and Fringe. Thus, the invention further relates to the use of an agent capable of downregulating the expression of *Delta* or *Serrate* proteins in the manufacture of a medicament for use in reversing bacteria infection or tumour-induced immunosuppression.

As discussed above, the invention also relates to the modification of Notch-protein expression or presentation on the cell membrane or signalling pathways. These have been shown to be involved in T-cell mediated responses that participate in the induction of tolerance (linked and/or bystander). Agents that enhance the presentation of a fully functional *Notch*-protein on the cell surface include matrix metalloproteinases such as the product of the Kuzbanian gene of Drosophila (Dkuz, Pan, D and Rubin, G.M. 1997 Cell 90: 271-280) and other ADAMALYSIN gene family members. Agents that reduce or interfere with its presentation as a fully functional cell membrane protein may include MMP inhibitors such as hydroxymate-based inhibitors.

The term "antigen presenting cell or the like" as used herein, is not intended to be limited to APCs. The skilled man will understand that any vehicle capable of presenting the desired Notch-ligand to the T cell population may be used, for the sake of convenience the term APCs is used to refer to all these. Thus examples of suitable APCs include dendritic cells, L cells, hybridomas, lymphomas, macrophages, B cells or synthetic APCs such as lipid membranes.

When the APCs are transfected with a gene capable of expressing a *Notch-*ligand, the transfection may be brought about by a virus such as a retrovirus or adenovirus, or by any other vehicle or method capable of delivering a gene to the cells. These include any vehicles or methods shown to be effective in gene therapy and include retroviruses, liposomes, electroporation, other viruses such as adenovirus, adeno-associated virus, herpes virus, vaccinia, calcium phosphate precipitated DNA, DEAE dextran assisted transfection, microinjection, polyethylene glycol, protein-DNA complexes.

Using such vehicles or methods alone or in combination it is possible to site-direct the gene delivery to a particular population of cells, thus enabling the method of the present invention to be performed in vivo. For example, a virus may be used in combination with liposomes in order to increase the efficiency of DNA uptake. The site specificity of the delivery may be achieved by the inclusion of specific proteins (eg a single chain antibody reactive with CD11c for dendritic cells/macrophages) in the viral coat or liposome.

Preferably, constructs through which expression of the the gene (eg serrate) is linked to antigen expression would be used. APCs (over)expressing Serrate would therefore also express high levels of the relevant antigen and so preferentially interact with T cells of the appropriate specificity.

A further embodiment of the present invention relates to a molecule comprising a *Notch*-ligand moiety operably linked to a T cell allergen or antigen moiety such that upon exposure to T cells both moieties are capable of binding to their respective sites. Such a molecule is capable of rendering an antigen/allergen specific T cell tolerant to the allergen or antigen upon which the allergen or antigen moiety is based, as the specificity required of the *Notch*-ligand moiety is provided by the close proximity of the allergen or antigen moiety.

The antigen or allergen moiety may be, for example, a synthetic MHC-peptide complex. That is a fragment of the MHC molecule bearing the antigen groove bearing an element of the antigen. Such complexes have been described in Altman JD et al Science 1996 274: 94-6.

In a further preferred embodiment, the compound is a fusion protein comprising a segment of *Notch* or *Notch* ligand extracellular domain and an immunoglobulin F_{c} segment, preferably IgGF_{c} or IgMF_{c} .

In all the above described embodiments of the present invention, it is preferable that the *Notch*-ligand is of the Serrate family of proteins or Delta family of proteins, derivatives, fragments or analogs thereof.

Diseased or infectious states that may be described as being mediated by T cells include any one or more of asthma, allergy, graft rejection, autoimmunity, tumour induced abberations to the T cell system and infectious diseases such as those caused by Plasmodium species, Microfilariae, Helminths, Mycobacteria, HIV, Cytomegalovirus, Pseudomonas, Toxoplasma, Echinococcus, Haemophilus influenza type B, measles, Hepatitis C or Toxicara. Thus, with use of the appropriate allergen or antigen, a *Notch-*ligand may be used in accordance with the present invention to treat the said disease or infection.

The invention also provides a method for detecting immune suppression induced by an invading organism. Such organisms may generate soluble forms of family members of *Serrate, Notch* and/or *Delta* or derivatives thereof or proteins that affect their expression in vivo, thus inducing infectious tolerance immunosuppression. The method comprises an assay for the presence of in vivo non-membrane bound *Serrate, Delta, Notch* or derivatives thereof and preferably comprises an antibody to *Serrate, Delta or Notch* or their derivatives.

Methods of use in screening assays for the detection of increased or decreased Notch, Delta/Serrate expression and/or processing include:
1. Delta/Serrate, Notch and Fringe expression being assessed following exposure of isolated cells to test compounds in culture using for example:
   (a) at the protein level by specific antibody staining using immunohistochemistry or flow cytometry.
   (b) at the RNA level by quantitative - reverse transcriptase-polymerase chain reaction (RT-PCR). RT-PCR may be performed using a control plasmid with in-built standards for measuring endogenous gene expression with primers specific for Notch 1 and Notch 2, Serrate 1 and Serrate 2, Delta 1 and Delta 2, Delta 3 and Fringe. This construct may be modified as new ligand members are identified.
   (c) at the functional level in cell adhesion assays.

Increased Delta/Serrate or Notch expression should lead to increased adhesion between cells expressing Notch and its ligands Delta/Serrate.Test cells will be exposed to a particular treatment in culture and radiolabelled or flourescein labelled target cells (transfected with Notch/Delta/Serrate protein) will be overlayed. Cell mixtures will be incubated at 37 degrees C for 2hr. Nonadherent cells will be washed away and the level of adherence measured by the level of radioactivity/immunofluorescence at the plate surface.

Using such methods it is possible to detect compounds or Notch-ligands that affect the expression or processing of a *Notch*-protein or *Notch*-ligand. The invention also relates to compounds, or *Notch*-ligands detectable by these assay methods.

The invention also includes an assay method comprising contacting (a) *Notch* protein and a ligand capable of binding to the *Notch* protein with (b) a compound; and determining if the compound affects binding of the ligand to the *Notch* protein preferably wherein the *Notch* protein is associated with a T cell.

The Notch-ligands of use in the present invention are preferably *Delta* or *Serrate* family member proteins or polypeptides or derivatives thereof. These are preferably obtained using standard techniques of recombinant technology well known to the person skilled in the art. Appropriate gene sequences for use to generate such compounds of the present invention may be obtained from publications such as WO97/01571, WO 96/27610 and WO 92/19734. The invention is not however in any way limited by the *Notch, Delta* and *Serrate* invention is not however in any way limited by the *Notch, Delta* and *Serrate* sequences disclosed in these publications. More preferably, such *Notch, Delta* or *Serrate* or family members, proteins or polypeptides or derivatives therefrom are fragments of the extracellular domains of *Notch, Delta* or *Serrate,* or family members or are derivatives of such fragments. As used herein, the term *"Notch* ligand" further includes any ligand or ligand family member that interacts with a *Notch* protein family member and includes the group of proteins referred to as "toporythmic proteins" i.e. the protein product of the *Delta, Serrate, Deltex* and *Enhancer of split* genes as well as other members of this gene family identifiable by virtue of the ability of their gene sequences to hybridize to, or their homology with Notch, Delta or Serrate proteins, or the ability of their genes to display phenotypic interactions.

Notch, Delta and Serrate were first described in Drosophila and therefore represent prototypic proteins of the Notch receptor and Notch-ligand family members respectively. Multiple Notch proteins and ligands have now been described in many invertebrate and vertebrate species but their nomenclature may differ from that used in the fly. For example Notch is a homolog of Lin 12 and Glp 1, Serrate/Delta are homologs of Jagged, Apxl and Lag-2.

Pharmaceutical formulations of the present invention may be formulated according to principles well known in the art. Thus the nature of the excipient and the amount of activity will depend upon the compound of the present invention which is to be formulated.

Preferably the pharmaceutical compositions are in unit dosage form.

Dosages of compounds of the present invention, to be administered to a patient in the form of a pharmaceutical formulation, could be determined by a suitable physician.

The preferred administration route of a formulation of the present invention is any one of the usual methods of administration including intra-muscular and intra-peritoneal, intravenous injection, intranasal inhalation, lung inhalation subcutaneous, intradermal, intra-articular, intrathecal, topical, and via the alimentary tract (for example, via the Peyers patches).

The term "derivative" as used herein, in relation to proteins or polypeptides of the present invention includes any substitution of, variation of, modification of, replacement of, deletion of or addition of one (or more) amino acid residues from or to the sequence providing that the resultant protein on polypeptide possesses the capability of modulating *Notch-Notch* ligand interactions.

The term "variant" as used herein, in relation to proteins or polypeptides of the present invention includes any substitution of, variation of, modification of, replacement of, deletion of or addition of one (or more) amino acid residues from or to the sequence providing that the resultant protein on polypeptide possesses the capability of modulating *Notch-Notch* ligand interactions.

The term "analog" as used herein, in relation to proteins or polypeptides of the present invention includes any peptidomimetic, that is, a chemical compound that possesses the capability of modulating *Notch-Notch* ligand interactions in a similar manner to the parent protein or polypeptide. These include compounds that may agonise or antagonise the expression or activity of a *Notch*-protein or *Notch*-ligand.

A compound may be considered to modulate *Notch-Notch* ligand interactions if it is capable of either inhibiting or enhancing the interaction of *Notch* with its ligands, preferably to an extent sufficient to provide therapeutic efficacy.

The expression "*Notch-Notch* ligand" as used herein means the interaction between a *Notch* family member and a ligand capable of binding to one or more such member.

The term therapy as used herein should be taken to encompass diagnostic and prophylactic applications.

The term "medical" includes human and veterinary applications.

As used herein, the terms protein and polypeptide may be assumed to be synonymous, protein merely being used in a general sense to indicate a relatively longer amino acid sequence than that present in a polypeptide.

The present invention will now be described by way of non-limiting example, with reference to the accompanying drawings, in which:
Figure 1 shows the results of in situ hybridisations carried out as described in Example 1 herein;
Figure 2 shows the results of the experiment described in Example 4.
Figure 3 shows the results of the experiment described in Example 5.
Figure 4 shows the results of the experiment described in Example 6.
Figure 5 shows the results of the experiment described in Example 7.
Figure 6 shows the results of the experiment described in Example 8.
Figure 7 shows the results of the experiment described in Example 9.
Figure 8a and 8b show the results of the experiment described in Example 10.
Figure 9 shows the results of the experiment described in Example 11.

### EXAMPLE 1

### Notch, Delta and Serrate are expressed in the peripheral immune system

Antisense RNA probes specific for *Notch 1, Delta 1* and *Serrate I* were synthesized and incorporated with digoxigenin labelled-UTP. Each probe was dissolved in hybridisation buffer, heated to 70°C for 5-10 minutes and added to TESPA coated slides containing 10mm sections of spleen or thymus that had been previously fixed with 4% paraformaldehyde + PBS. Slides were hybridized overnight at 65°C. The following day, slides were washed twice at 65°C. and twice at room temperature (RT) with 1xSSC/50% Formamide/0.1%Tween 20. Slides were washed twice with 0.1M maleic acid/0.15M NaCl/0.1% tween 20 pH 7.5 (MABT) buffer at RT and then blocked for 2 h with MABT + 20% goat serum + 296 Boehringer blocking reagent (BBR). Slides were incubated overnight at RT with anti-digoxidenin F_{ab} fragments. After four washes with MABT slides were washed a further two times in alkaline substrate buffer. The presence of bound anti-sense RNA probes was detected by incubating slides in substrate buffer containing NBT + BCIP in the dark. Slides were conterstained with haemotoxylin and mounted in Depx mounting medium.

Results: The results of these hybridisations are shown in Figure 1, that shows that in a spleen from a 3 month old mouse, Delta and Serrate are expressed by isolated cells in the periarteriolar sheath and not in the germinal centre(gc). Notch is expressed in many cells again in the periarteriolar sheath.

### EXAMPLE 2

### Production of Delta-Fc fusion protein

The pIG-1 [D. Simmons, "Cloning cell surface molecules by transient surface expression in mammalian cells" pp 93-128, Cellular Interactions in Development Ed. D. Hartley, pub. Ox. Uni. Press (1993)] expression vector allows production of a fusion protein that contains the extracellular portion of Delta 1 linked to the human IgG1-F_{c} domain. A restriction enzyme fragment that contained only the extracellular domain of the Delta 1 protein was cloned into the pIG-1 vector. The resultant plasmid was tranformed into *E. Coli* MC 1061, and grown in SOB containing 10µg/ml tetracycline/ampicillin. Purified vector was used to transfect COS cells *in vitro.* COS cells were grown to 50-75% confluency and transfected with 10 µg of plasmid DNA per dish by DEAE-dextran method. At 24 h post transfection the culture medium was replaced with culture medium containing 1% FCS and cells were cultured for a further 3-6 days *in vitro.* Cells were spun for 5mins/5000 rpm to pellet cells and debris, the supernatant was removed and stored until required. The Delta-Fc fusion was purified from culture supernatants by adding 2ml of 50% slurry of protein a Sepharose (Phamacia) and rotated overnight at 4°C. Sepharose beads were isolated by passing culture supernatants through a 0.45 mm filter, washed and transferred to a 10 ml plastic column. The Delta-Fc fusion construct was eluted with 2 ml of elution buffer pH 4.0. The eluate was neutralised by the addition of 1M Tris base.

### EXAMPLE 3

### Examples of models in which the Notch-ligand signalling pathway may be investigated

Peripheral tolerance to self antigens can be analysed in T cell receptor (TCR) transgenic mice in which the TCR ligand is expressed as a self antigen only in the periphery. Peripheral tolerance to transplantation antigens can be induced in several ways including recipient pre-treatment with T cell antibodies or blockade of costimulation. It is thereby possible to demonstrate both linked suppression and infectious tolerance. Peripheral tolerance to allergens may be induced by the intranasal delivery of allergen derived peptides. The expression of *Notch-Notch* ligands is measured on cells recruited into the airways and/or lymphoid tissues following allergen inhalation and modifications in tolerance demonstrated. Furthermore, in experimental models of infections with infectious agents, the expression of *Notch-Notch* ligands can be measured on the organism (pathogen) and immunocompetent cells in the host.

### EXAMPLE 4

### Delta expressing hybridomas can inhibit the responses of antigen primed lymphocytes.

Mice were immunised with a synthetic peptide containing an immunodominant epitope of the house dust mite allergen (HDM), Der p1 (p110-31), or with ovalbumin (OVA, hen egg white protein). One week later the lymph node cells (LNCs) were removed and cell suspensions made. Lymph nodes from animals immunised with different antigens were kept separate. These cells are referred to as primed LNCs.

T cell hybridomas were transfected with either full length Delta or a control plasmid, such that delta was expressed as a membrane protein. After two days in culture the hybridomas were irradiated to prevent them from proliferating or from producing cytokines. Therefore, the only response which was measured in the assay comes from the lymph node cells alone.

The irradiated hybridomas were added in increasing numbers to cultures containing the primed LNCs. The appropriate antigen (i.e. p110-131 or OVA) was added and the cells cultured for 24 hours. Supernatent fluids were then collected and assayed for IL-2 (a major T cell growth factor) content. Proliferative responses of the lymph node cells after 72 hours were also measured.

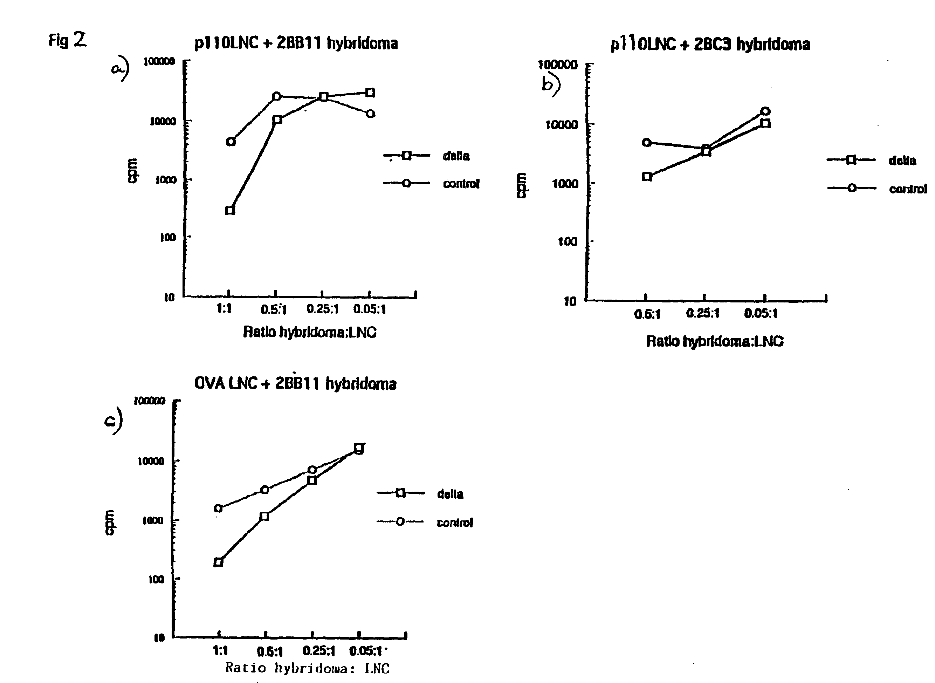

Results: Lymph node cells cultured in the presence of irradiated hybridomas that expressed a control plasmid still proliferated as shown in Figure 2 and secreted IL-2 when stimulated in culture with the appropriate antigen. Their responsiveness was maintained at a ratio of 1:1 LNC:hybridoma. In contrast, the proliferative response and production of IL-2 by lymph node cells was reduced by at least 88 % when cultured in the presence of hybridomas expressing full length Delta (at a ratio of 1:1) with the appropriate antigen. Hybridomas transfected with control virus (open circles), delta virus (open squares). Figure 2 shows the data presented as counts per minute (cpm) ³H-Tdr incorporation 72 hours after the beginning of culture. Cpm of lymph node cells (LNC) cultured with hybridomas expressing delta or control constructs. Total numbers of cells/well = 4 x 10⁵ (i.e the number of LNCs varies according to the ratio of hybridomas to LNC, so the cpm will vary). p110-131 LNC are cells primed with Der p1 (p110-131), OVA LNC are cells primed with OVA. 2BB11 and 2BC3 are two different Der p1 reactive hybridomas.

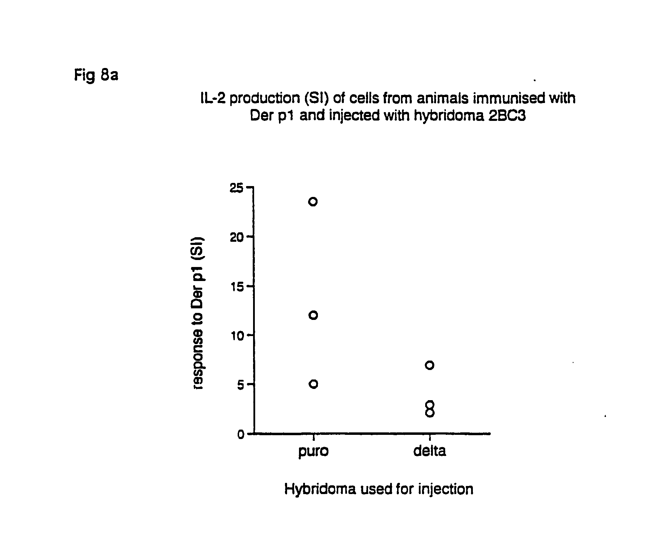

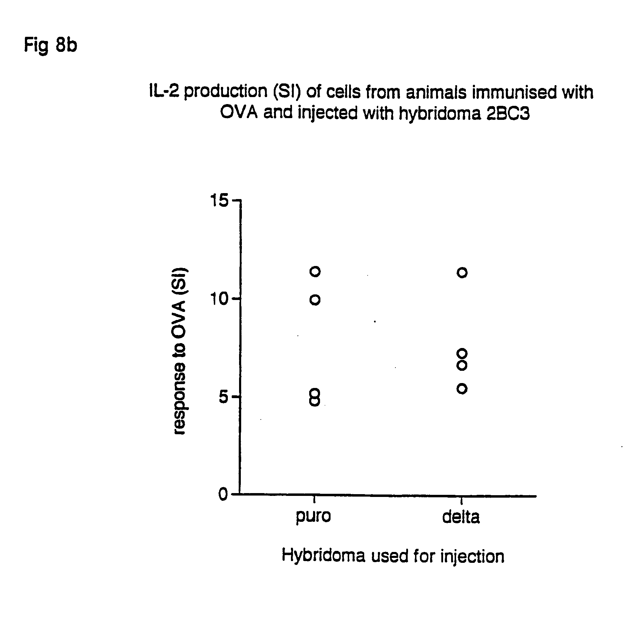

These data show that inhibition of responses by Delta expressing T cells can be delivered in trans. Although in this culture system Delta expressing T hybridomas specific for Derp 1 were able to inhibit the response of OVA primed T cells, this apparent lack of specificity appears to be due to the close proximity of cells forced by the culture system. Indeed, the data shown in Figures 8a and 8b show that in animals the delta expressing hybridoma must share antigen specificity with the immunogen for there to be an modulating effect on the immune response to that immunogen. In this case it appears that the delta expressing T cells can only be brought into proximity with the responding T cells if they recognise the same antigen on the same APC.

### EXAMPLE 5

### Serrate expressing Dendritic cells prevent antigen priming of T lymphocytes.

Dendritic cells (DCs) are the primary antigen presenting cell in the immune system and are critical for stimulating T cell responses. DCs were obtained from the spleen and transfected with either a retrovirus allowing expression of the full length Serrate protein or a control retrovirus. The DCs were also pulsed with the HDM peptide p110-131 for 3 hours in vitro at 37°C. The DCs were then washed and used to immunise naive mice subcutaneously using 10⁵ cells/mouse. After 7 days the draining LNCs were recovered and restimulated in culture with peptide at 4x10⁵ cells/well. Since the mice were only immunised with peptide-pulsed DCs this gives us a measure of the ability of these cells to prime an immune response.

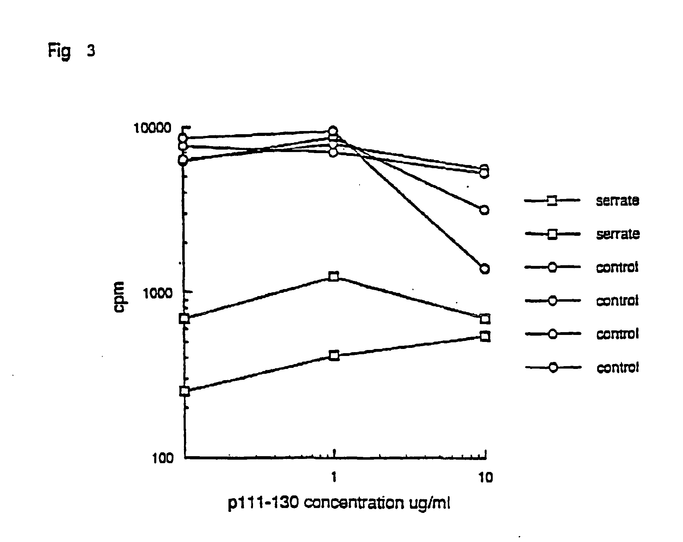

Results: Figure 3 shows the data presented as cpm of LNCs 72 hours after culture from animals immunised with control transfected (open circles) or serrated transfected (open squares) dendritic cells (DCs).

Immunisation of mice with DCs expressing Serrate resulted in a 10 fold decrease in the number of cells recovered from lymph nodes when compared to immunisation with control DCs. We further found that LNCs from mice immunised with DCs+Serrate failed to proliferate (93% reduction on control values, Figure 3) or secrete IL-2 when compared to cells from mice immunised with control DCs.

### EXAMPLE 6

### Delta expressing T cell hybridomas are able to inhibit the development of immunity to Der p 1 antigen in animals.

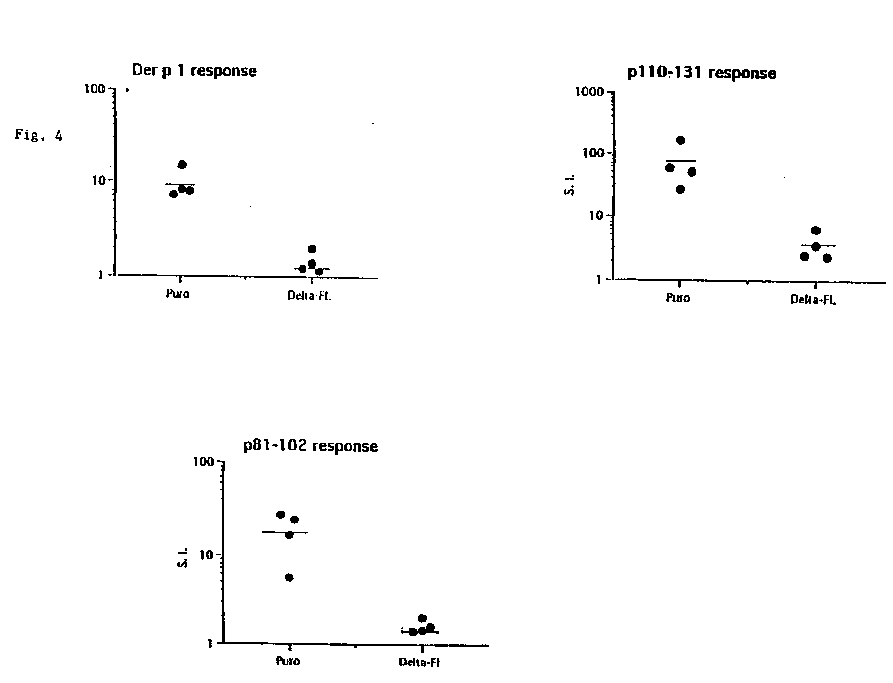

T cell hybridomas (reactive with Der p 1) were transfected with a retrovirus containing mouse Delta such that Delta was expressed on the cell surface or with a control retrovirus. C57 BL mice were injected with 10 million irradiated hybridomas i.p. and immunised with 50 microgram Der p 1 emulsified in Complete Freunds Adjuvant (CFA) sub-cutaneously. After 7 days the draining lymph node cells were collected and cultured at 4 x 10⁵ cells/well with Der p 1 (10 microgram/ml), peptide 110-131 of Der p 1 (10 microgram/ml), or peptide 81-102 of Der p 1 (10 microgram/ml). Cultures were incubated at 37°C for 72 hours and tritiated thymidine added for the final 8 hours of culture. Results of proliferation assays of cells from animals injected with control transfected (puro) or Delta transfected (Delta-FL) are shown in Figure 4.

Results: LNC from animals injected with control virus transfected hybridomas produced high levels of IL-2 and proliferated in culture in the presence of Der p 1, peptide 110-131 or peptide 81-102. In contrast, cells from animals injected with Delta expressing hybridomas made no response to any of the Der p 1 antigens (Fig. 4).

### EXAMPLE 7

### Delta expressing human T cells can block the response of normal T cells.

An influenza-reactive human T cell clone (HA1.7) was transfected with mouse Delta using a retroviral construct to allow cell surface expression of the Delta protein. Mixing of this cell population with normal HA1.7 prevented subsequent reactivity of these normal HA1.7 with peptide HA306-318 and antigen presenting cells. 5 x 10⁵ HA1.7 were mixed with 1 x 10⁶ irradiated DRB1*0101 pripheral blood mononuclear cells (PBMC) + 1 microgram HA306-318 and cultured at 37°C. 6 hours later 5% lymphocult (IL-2 containing medium) was added in a total volume of 1ml. 24 hours after the initiation of culture Delta or control retrovirus or nothing was added. 7 days after the start of culture, cells were harvested, washed and the transfected cells irradiated. The transfected cells were mixed at a ratio of 2:1 with untreated HA1.7 and cultured for 2 days. Mixed cultures were then harvested, washed and plated out using 2 x 10⁴ viable cells/well together with
a) 2.5 x 10⁴ DRB1*0101 PBMCs (medium)
b) 2.5 x 10⁴ DRB1*0101 PBMCs + peptide (Ag + APC)
c) 5% lymphocult (IL-2)

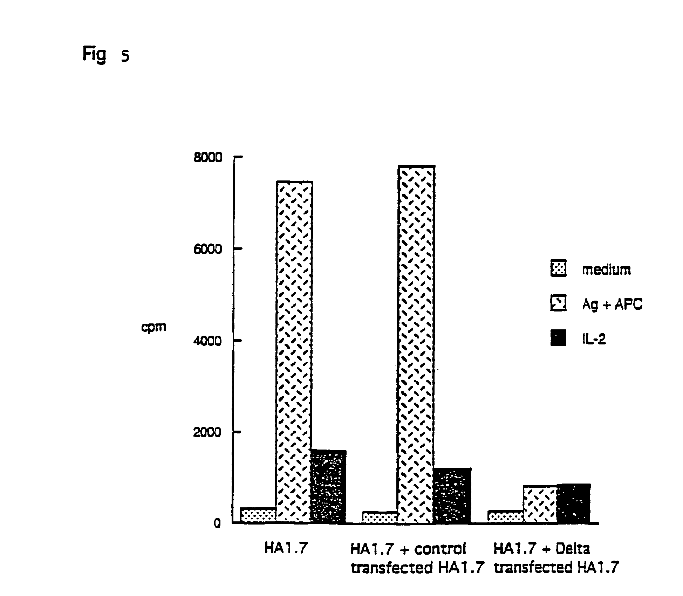

Cells were harvested after 68 hours with the addition of tritiated thymidine for the final 8 hours. The results are shown in Figure 5.
Results: Following culture alone or with control virus transfected HA1.7, untreated HA1.7 responds well to peptide + antigen presenting cells. Incubation with Delta transfected irradiated HA1.7 completely prevents the response of untreated HA1.7 to antigen + APC. However, such cells respond as well as untreated or control virus incubated HA1.7 to IL-2, indicating that they are not simply unable to proliferate (Fig. 5).

### EXAMPLE 8

### Serrate expression by antigen presenting cells prevents T cell responses.

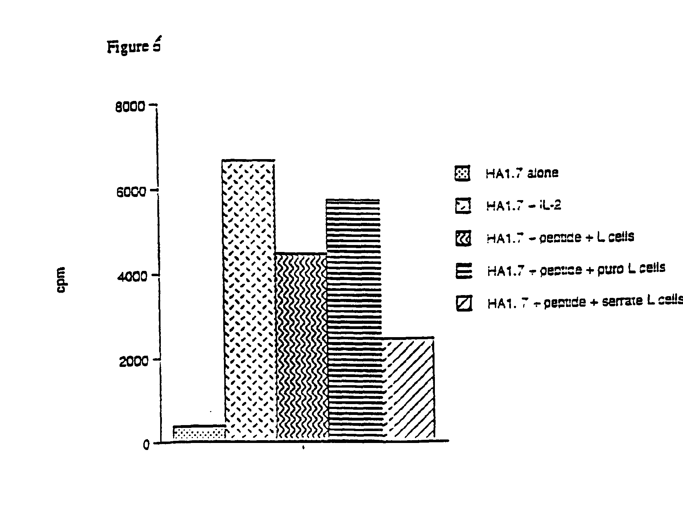

Clone HA1.7 was mixed with peptide HA306-318 (1.0 microgram/ml) in the presence of L cells expressing HLA-DRB1*0101(as antigen presenting cells), using 2x10⁴ of each cell type. The L cells were transfected with either control (puro) or serrate (serrate L cells) expressing retrovirus. The proliferative response was measured after 72 hours following addition of tritiated thymidine for the last 8 hours of culture. Results are shown in Figure 6 for HA1.7 cultures:
a) alone 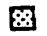
b) +IL-2 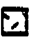
c) + peptide + DRB1*0101-L cells 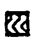
d) + peptide + DRB1*0101-L cells transfected with control virus 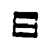
e) + peptide + DRB1*0101-L cells transfected with serrate virus 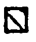

Results: HA1.7 stimulated by serrate expressing L cells responded poorly to antigen when compared with those stimulated by control transfected L cells (Fig. 6).

### EXAMPLE 9

### Serrate expressing antigen presenting cells induce regulatory T cells.

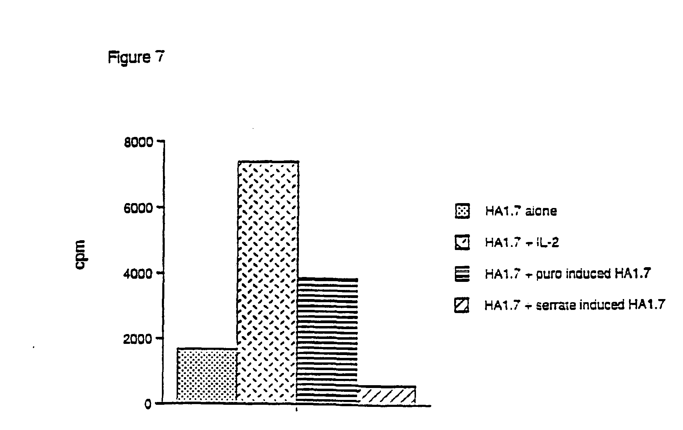

Clone HA1.7 was mixed with peptide HA306-318 and L cells (expressing DRB1*0101 as antigen presenting cells) in the presence of 2% IL-2. The L cells were transfected with either control or serrate expressing retrovirus. After 7 days in culture, the HA1.7 were harvested washed and irradiated before being mixed with fresh HA1.7 (using 2x10⁴ each population). Cells were cultured for a further 2 days before being stimulated with peptide (1.0 microgram/ml) + normal antigen presenting cells (DRB1*0101 PBMCs). The proliferative response was measured after 72 hours following addition of tritiated thymidine for the last 8 hours of culture. The results are shown in Figure 7 for fresh HA1.7 cultured:
a) alone 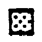
b) + IL-2 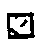
c) + control virus induced HA1.7, then peptide + DRB1*0101 PBMC 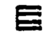
d) + serrate virus induced HA1.7, then peptide + DRB1*0101 PBMC 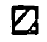

Results: HA1.7 induced by serrate expressing L cells (Serrate induced HA1.7) prevented the response of normal HA1.7 to a normal antigenic stimulus (Fig. 7). This shows the ability of cells tolerised by exposure to serrate, to pass on their tolerance to a naive cell population (infectious/bystander tolerance).

### EXAMPLE 10

### The regulation induced by delta expressing T cells is antigen specific.

Mice were injected with 10⁷ T cell hybridoma cells transduced with either delta or control retrovirus. The hybridoma was 2BC3 which has specificity for peptide 110-131 of Der p1. At the same time, animals received either Der p 1 or ovalbumin emulsified in complete Freund's adjuvant. 7 days later, lymph node cells were removed and 4x10⁵ cells were cultured in the presence of Der p1 or ovalbumin (using the same antigen that they had already been immunised with).

The production of IL-2 was measured 24 hours after the start of culture. Stimulation indexes for IL-2 production are shown in Figures 8a (responses of animals immunised with Der pl) and 8b (responses of animals immunised with OVA) for animals injected with hybridomas transfected with control (puro) or Delta (delta) retrovirus.

Results: The response to Der p1 in animals injected with delta expressing 2BC3 hybridomas was virtually completely abolished whereas the response to ovalbumin was unaffected. These data are shown in Fig. 8a and 8b where each dot represents the IL-2 production by an individual mouse to antigen as a stimulation index (SI) of control responses (no antigen added).

### EXAMPLE 11

### Delta is expressed on T cells during the induction of tolerance

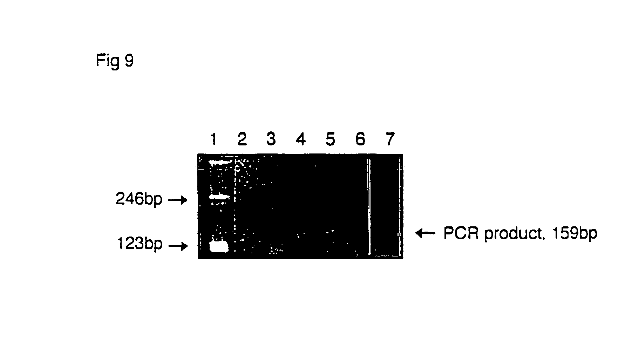

HA1.7 was cultured in the presence of peptide HA306-318 (50 µg/ml) in the absence of APCs. Such conditions result in a state of tolerance in the HA1.7. After 0,30, 120, 240 or 360 minutes, cells (1.5x10⁶) were harvested , pelleted and frozen. RNA was prepared from cell pellets by homogenisation in guanidium thiocyanate solution followed by CsCl density centrifugation. 1 µg RNA was converted into cDNA using an oligo dT primer. Of the resultant cDNA, 1/20th was used in PCR (40 cycles) using primers specific for the human delta homolog. PCR samples were analysed by gel electrophoresis (Figure 9) wherein lane 1, marker, lane 2, t=0, lane 3, t=30min, lane 4, t=120min, lane 5, t=240 min, lane 6,t=360min and lane 7 is the negative control.

Results.: Resting HA1.7 did not express Delta mRNA, but transcripts appeared within 2 hours of initiation of the tolerisation protocol.

### EXAMPLE 12

### Analysis of Notch 1, Serrate 1 and Delta 1 expression during the induction of tolerance by immunohistology and in site hybridisation.

C57 BL/6J mice were treated intranasally with either 100 microgram Der p 1 peptide 110-131 or a control solution (phosphate buffered saline, PBS) on three consecutive days. Intranasal adminstration of antigen in this way is known to induce tolerance to the antigen. Some animals were then rested for 2 weeks before being rechallenged with antigen by injection of 50µg Der p1/CFA subcutaneously into the base of the tail. The superficial lymph nodes and spleens of animals were harvested at various time points thereafter (d0 being the first day of intranasal treatment or antigen rechallenge) and processed for immunohistology or in situ hybridisation. For immunohistology, tissues were frozen and 3µm sections cut, fixed in ice cold acetone and stained with polyclonal antibodies specific for Notch 1 and Serrate 1. Bound antibody was detected using a horseradish peroxidase conjugated goat anti-rabbit antibody developed with diaminobenzidine as the substrate. Delta 1 specific antibodies were not available for the study. For in situ hybridisation, frozen tissues were sectioned and fixed in 4% paraformaldehyde. Sections were hybridised with digoxigenin coupled antisense RNA probes specific for Notch 1, Serrate 1 and Delta 1 at 65°C. Bound probe was detected by alkaline phosphatase conjugated goat anti-digoxigenin antibody developed using NBT and BCIP as the substrate. Data shown in tables 1 and 2 are for immunohistology and in situ hybridisation respectively. Data represent the analysis of tissues from 5 separate mice after intranasal peptide alone (PBS/p110-131 primary) or intranasal and subcutaneous antigen (PBS/p110-131 rechallenge.
+ weak staining, ++ moderate staining, +++ stong staining.
Results:
Basal levels of Notch, Delta and Serrate are expressed in control mice receiving only PBS. Mice dosed intranasally with PBS and subcutaneously with antigen showed a moderate increase in expression of all three molecules within 8 days of rechallenge. Animals given either intranasal peptide alone or intranasal peptide followed by antigen rechallenge showed the same pattern of increased expression of Notch Delta and Serrate which was more rapid and greater than in control mice.

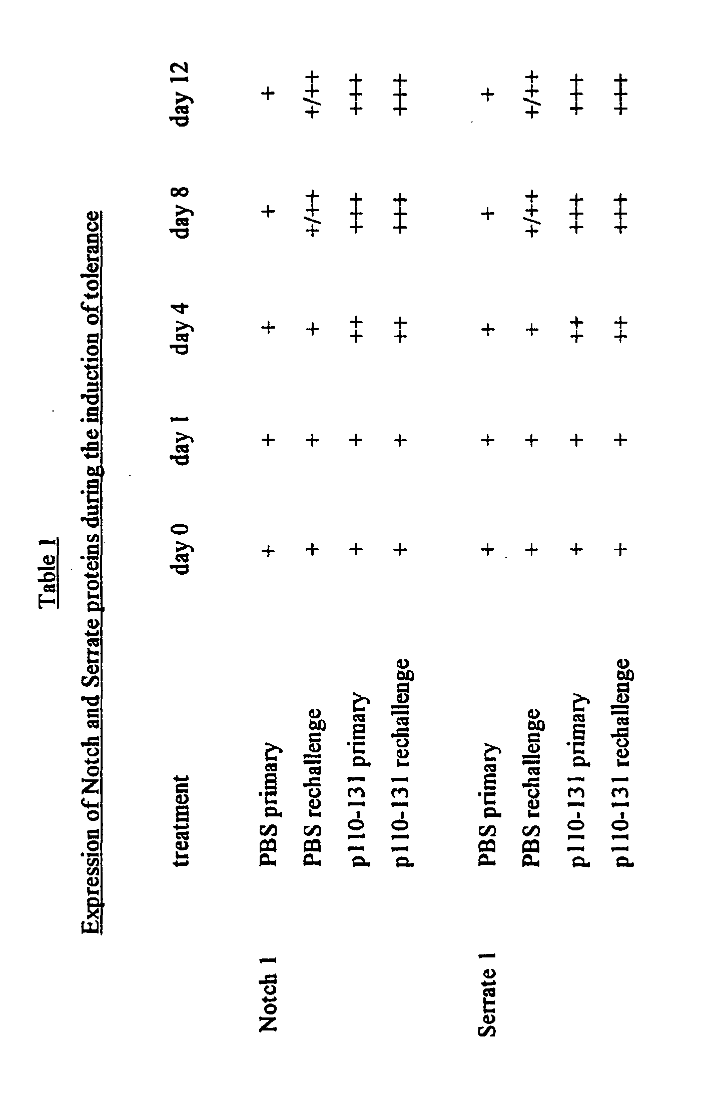

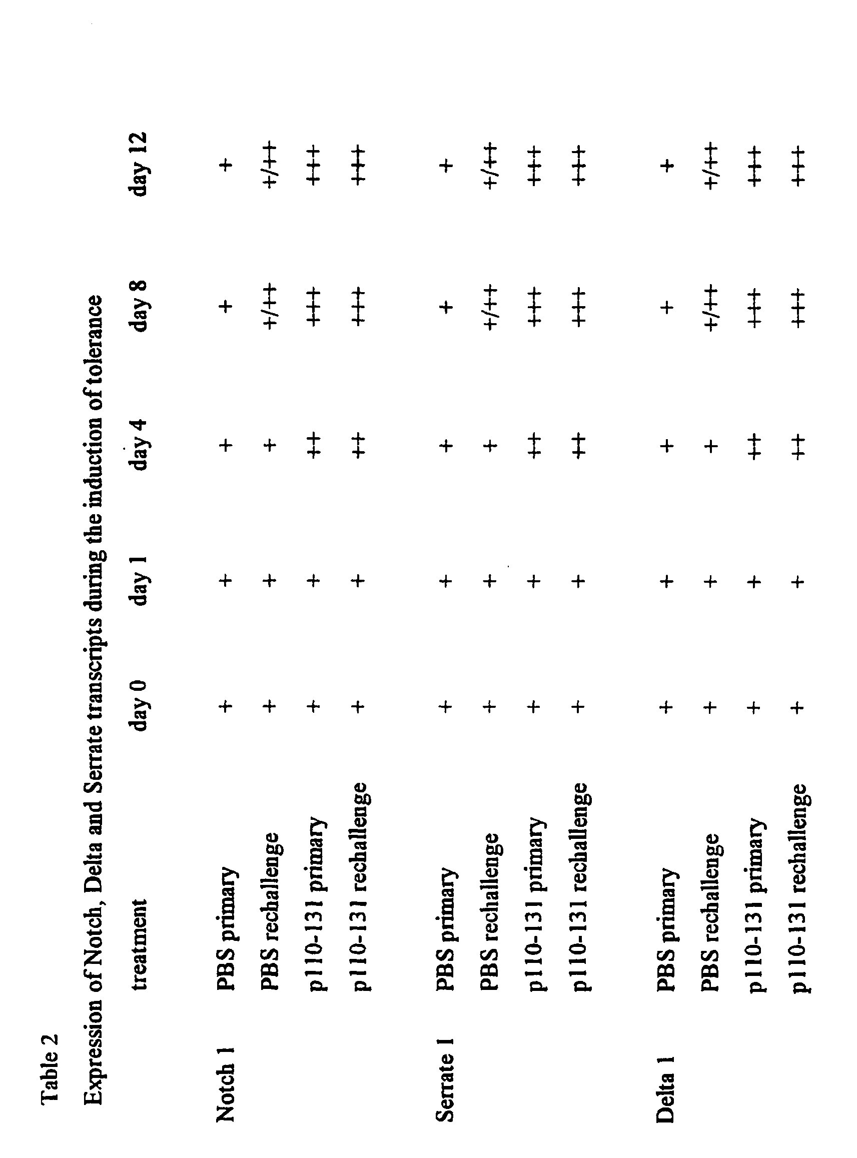

Other modifications of the present invention will be apparent to those skilled in the present art.

## Claims

1. Use of a *Notch*-ligand in the manufacture of a medicament for use in immunotherapy.

2. Use according to claim 1, wherein the immunotherapy involves the treatment of a T-cell mediated disease or infection.

3. Use according to claim 2, wherein the T cell mediated disease or infection is due to of any one or more of allergy, autoimmunity, graft rejection, tumour induced abberations to the T cell system and infectious diseases such as those caused by Plasmodium species, Microfilariae, Helminths, Mycobacteria, HIV, Cytomegalovirus, Pseudomonas, Toxoplasma, Echinococcus, Haemophilus influenza type B, measles, Hepatitis C or Toxicara.

4. Use according to any of claims 1 to 3, wherein the *Notch*-ligand is selected from Serrate, Delta or fragments, derivatives or analogs thereof.

5. A *Notch-ligand,* or fragments, derivatives or analogs thereof, for use in affecting linked suppression.

6. A *Notch-ligand,* or fragments, derivatives or analogs thereof, for use in affecting infectious tolerance.

7. A method of tolerising T cells to an allergen or antigen comprising incubating/exposing said T cells to antigen presenting cells expressing or overexpressing a *Notch*-ligand; in the presence of said allergen or antigen.

8. A method according to claim 7, wherein the (over)expression of the *Notch-*ligand is due to the APC being transfected with a virus capable of expressing said ligand.

9. A method according to claim 7, wherein the (over)expression of the *Notch-*ligand is due to the APC being stimulated by an agent that up-regulates expression of a *Notch*-ligand expressing gene.

10. A method according to claim 9, wherein the agent is selected from Noggin, Chordin, Follistatin, Xnr3, fibroblast growth factors or derivatives or fragments thereof.

11. A method according to claim 7, 8,9 or 10 wherein the APC is selected from dendritic cells, L cells, hybridomas, lymphomas, macrophages, B cells or synthetic APCs such as lipid membranes.

12. A method according to any of claims 7 to 11, wherein the *Notch*-ligand is selected from Serrate, Delta or fragments, derivatives or analogs thereof.

13. A molecule comprising a *Notch*-ligand moiety operably linked to a T cell allergen or antigen moiety such that upon exposure to T cells both moieties are capable of binding to their respective sites.

14. A molecule according to claim 12 or 13, wherein the *Notch*-ligand moiety is selected from *Serrate, Delta* or fragments, derivatives or analogs thereof.

15. A fusion protein comprising a segment of a *Notch* ligand extracellular domain and an immunoglobulin F_{c} segment.

16. A fusion protein according to claim 15 wherein the *Notch* ligand extracellular domain is derived from *Notch, Delta* or *Serrate.*

17. A fusion protein according to claim 15 or 16 wherein the F_{c} segment is IgGF_{c} or IgMF_{c}

18. A kit comprising immobilised *Notch* or family members to allow measurement of soluble *Notch* ligands originating from pathogens, transplanted grafts or cells of the host.

19. An assay method comprising contacting (a) a *Notch* protein and a ligand capable of binding to the *Notch* protein with (b) a compound; and determining if the compound affects binding of the ligand to the *Notch* protein.

20. A ligand capable of binding to *Delta* or *Serrate* proteins or their derivatives expressed on or secreted by pathogenic organisms tumours or transplanted grafts, for use in medical therapy.

21. An assay method comprising determining the in vivo level of soluble free *Serrate, Notch* or *Delta* family members or forms thereof.

22. An assay for detecting the affect of a compound on the expression or processing of functional Notch-protein or *Notch* ligand comprising contacting cells expressing *Notch*-protein or *Notch* ligand with a compound; and monitoring an increase or decrease in expression or processing of said protein or ligand.

23. Compounds, *Notch*-legands, derivatives, fragments or analogs thereof, detectable by the assay of claim 22.

24. Use of an agent capable of downregulating the expression of *Delta* or *Serrate* proteins or reducing its presentation as a cell membrane protein in the manufacture of a medicament for use in reversing bacteria, infection or tumour-induced immunosuppmssion.

25. Use according to claim 24, wherein the agent is a Toll protein or bone morphogenetic protein.

26. Use of a *Notch*-ligand in the modulation of the expression of a functional *Notch*-protein or *Notch* signalling pathways.
